# EUROPEAN PATENT APPLICATION

(11) **EP 3 970 689 A1**
(43) Date of publication of application: **23.03.2022**
(21) Application number: 20805185.4
(22) Date of filing: 13.05.2020
(51) Int. Cl.: A61K 8/02, A61Q 1/00, A61Q 19/00

(54) **THIN FILM AND TRANSFER SHEET**

(30) Priority: 16.05.2019 JP 2019093147
(71) Applicant: TOPPAN INC., Tokyo 110-0016 (JP)
(72) Inventor: WATANABE Manabu, Tokyo 110-0016 (JP)
(74) Representative: TBK
(86) International application number: PCT/JP2020/019118
(87) International publication number: WO 2020/230817

(57) **Abstract**

A thin film has an average thickness of 0.1 µm or more and 5.0 µm or less. The thin film has a first surface, and a second surface located on a side opposite to that on which the first surface is located, and the first surface and the second surface each have a concavo-convex structure. The thin film includes an element, which is a portion where a projection is located on the first surface and a corresponding recess is located on the second surface. The thin film includes a plurality of the elements.

## Description

### [Technical Field]

The present invention relates to a thin film and a transfer sheet for adhering the thin film to an adherend.

### [Background Art]

Ultra-thin films having a thickness of the order of several nanometers to several micrometers can conform to a variety of shapes due to their high flexibility, and can adhere to a surface of biological organs without adhesives or pressure-sensitive adhesives. Accordingly, such films have been used for adhesion to an adherend such as organs or skin. For example, PTL 1 proposes adhesion of such films to the skin for assisting in skin care or makeup.

### [Citation List]

### [Patent Literature]

[PTL 1] WO 2014/058060

### [Summary of the Invention]

### [Technical Problem]

In the thin films described above, the surface of the film tends to appear colored and shiny due to interference of light reflected on the front surface of the film and light reflected on the rear surface of the film. Such films are often regarded as having poor appearance, which is not desirable especially in cosmetic applications since the adhered portion of the film looks shiny, that is, glossy due to sebum secretion. The colored appearance due to interference of reflected light can be suppressed by, for example, roughening the surface of the film. However, as described above, since the film is extremely thin to achieve high conformability to a surface shape of an adherend, it is difficult to form a concavo-convex structure with a sufficient height on a surface of the film while maintaining the characteristics due to a small film thickness. Further, various active ingredients can be added to the films for imparting functions to the films. However, the amount of active ingredients that can be added is limited in order to maintain the shape conformability, which is a characteristic due to a small film thickness.

An object of the present invention is to provide a thin film and a transfer sheet, in which the thin film is capable of preventing a decrease in the characteristics due to a small film thickness while improving other functions.

### [Solution to Problem]

A thin film for solving the above problem includes: a first surface; and a second surface located on a side of the thin film opposite to that on which the first surface is located, wherein the thin film has an average thickness of 0.1 µm or more and 5.0 µm or less, the first surface and the second surface each have a concavo-convex structure, the thin film includes an element, which is a portion where a projection is located on the first surface and a corresponding recess is located on the second surface, and the thin film includes a plurality of the elements.

With this configuration, compared with a case where a concavo-convex surface is formed only on the first surface, portions of the thin film where the projections are located on the first surface are prevented from increasing in film thickness. Accordingly, the thin film can have a concavo-convex structure on the surfaces while preventing a decrease in the characteristics due to the small film thickness. Due to the concavo-convex structure provided on the surface, light can be scattered and the surface area can be increased. As a result, the functions of the thin film can be improved. For example, it is possible to suppress a colored or shiny appearance of the thin film due to interference of light while preventing a decrease in shape conformability of the thin film, and improve a soft focus effect.

In the above configuration, a cross-section of the thin film may include: a first portion, which is a portion where a top of a projection is located on the first surface and a corresponding bottom of a recess is located on the second surface; and a second portion, which is a portion where a bottom of a recess is located on the first surface and a corresponding top of a projection is located on the second surface, the first portion and the second portion being alternately arranged.

With this configuration, the thin film itself is repeatedly curved to form a concavo-convex structure on the first surface and the second surface, whereby the thin film has a concavo-convex structure on the surfaces while preventing an increase in film thickness in a wide area of the thin film. Therefore, it is possible to obtain an effect produced by a surface having a concavo-convex structure in a wide area of the thin film while preventing a decrease in the characteristics due to the small film thickness.

In the above configuration, an interval between the elements adjacent to each other may be 100 µm or less, an average height of the plurality of elements may be 2.0 µm or more and 50.0 µm or less, and a ratio of the average height to an average width of the plurality of elements may be 1.5 or less.

With this configuration, the shape conformability, strength, and soft focus effect of the thin film can be favorably obtained, and the thin film can be suitably prevented from appearing colored or shiny due to interference of light.

In the above configuration, when an area of the thin film in plan view as viewed in a direction perpendicular to a plane parallel to an extending direction of the thin film is defined as a reference area, the first surface may have a surface area of 1.1 times or more and 3.0 times or less the reference area.

With this configuration, the thin film has good strength, and an effect produced by an increase in the surface area of the thin film can be favorably obtained.

In the above configuration, the thin film may be made of a material having biocompatibility or biodegradability.

With this configuration, the thin film has increased suitability for use in applications in which the thin film is adhered to biological tissues and for use as a substrate for cell culture.

A transfer sheet for solving the above problem is a transfer sheet including: the thin film described above; and a support substrate that supports the thin film.

With this configuration, since the thin film is supported by the support substrate, the thin film is prevented from deforming, which improves the ease of handling of the thin film.

In the above configuration, the support substrate may be one of a woven fabric, a non-woven fabric, a sheet of paper, and a mesh sheet.

With this configuration, the support substrate is permeable to liquid. Accordingly, when the support substrate is exposed to liquid such as water, the liquid is easily distributed into the support substrate. Therefore, when the support substrate is configured to deform when exposed to liquid, the deformation is promoted and the releasability of the support substrate from the thin film is suitably improved.

### [Advantageous Effects of the Invention]

According to the present invention, it is possible to prevent a decrease in the characteristics due to a small film thickness while improving other functions.

### [Brief Description of the Drawings]

Fig. 1 is a perspective view illustrating an example of a structure of a thin film according to an embodiment of the thin film.
Fig. 2 is a view illustrating an example of a perspective structure of a thin film according to an embodiment.
Fig. 3 is a view illustrating an example of a perspective structure of a thin film according to an embodiment.
Fig. 4 is a view illustrating an example of a cross-sectional structure of a thin film according to an embodiment.
Fig. 5 is a view illustrating an example of a cross-sectional structure of a thin film according to an embodiment.
Fig. 6 is a view illustrating an example of a cross-sectional structure of a thin film according to an embodiment.
Fig. 7 is a view illustrating an example of a cross-sectional structure of a thin film according to an embodiment.
Fig. 8 is a view illustrating an example of a planar structure of a thin film according to an embodiment.
Fig. 9 is a view illustrating an example of a planar structure of a thin film according to an embodiment.
Fig. 10 is a view illustrating an example of a planar structure of a thin film according to an embodiment.
Fig. 11 is a view illustrating an example of a planar structure of a transfer sheet according to an embodiment of the transfer sheet.
Fig. 12 is a view illustrating an example of a cross-sectional structure of a transfer sheet according to an embodiment.
Fig. 13 is a view illustrating an example of a cross-sectional structure of a transfer sheet according to an embodiment.

### [Description of the Embodiments]

With reference to the drawings, an embodiment of a thin film and a transfer sheet will be described below.

When used, a thin film of the present embodiment is adhered to biological tissues. Specifically, applications of the thin film include cosmetic applications in which a thin film is adhered to the skin to assist in skin care or makeup, applications in which a thin film is adhered to organs or cells to prevent tissue adhesion during surgery, and applications in which a thin film is adhered to wounds in the skin or organs to treat the wounds. The biological tissues refer to animal tissues, which include epithelial tissues such as skin, connective tissues such as cartilage and bone, muscle tissues, and nerve tissues. Further, the thin film of the present embodiment may also be used as a substrate for cell culture.

In cosmetic applications, the thin film may be adhered to the skin after cosmetics are applied to the skin, or may be adhered to the skin before cosmetics are applied to the skin. Alternatively, cosmetics may be applied to the film after the thin film is adhered to the skin.

### [Configuration of Thin Film]

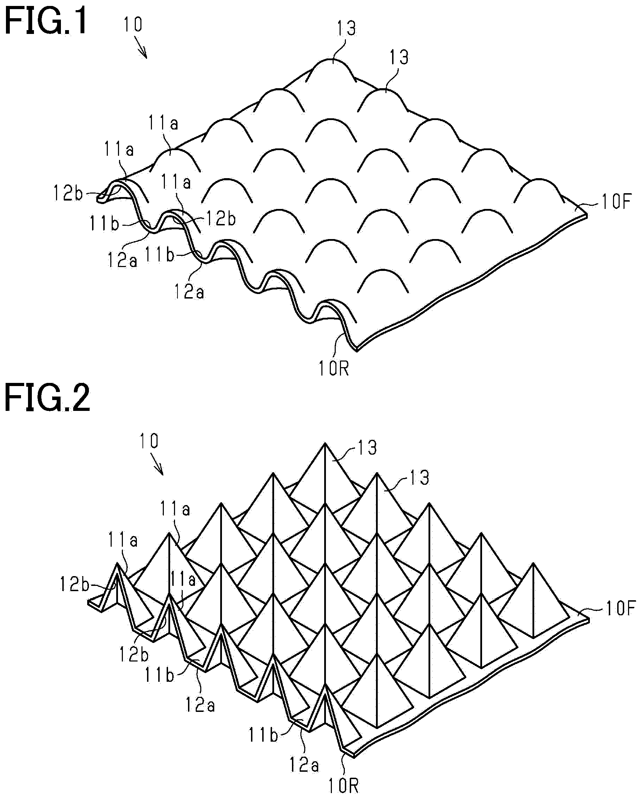

Figs. 1 to 3 illustrate examples of a perspective structure of a thin film 10. The thin film 10 has a first surface 10F and a second surface 10R located on a side opposite to that on which the first surface 10F is located.

The first surface 10F has a concavo-convex structure. Specifically, the first surface 10F has a plurality of projections 11a protruding from the first surface 10F. Portions between the adjacent projections 11a or boundaries between the adjacent projections 11a constitute recesses 11b that are recessed from the first surface 10F. The size of the plurality of projections 11a may be constant or may not be constant. Further, the plurality of proj ections 11a may be arranged regularly or irregularly.

The second surface 10R has a concavo-convex structure having an inverted shape of the concavo-convex structure of the first surface 10F. That is, portions on the first surface 10F where the projections 11a are located correspond to portions on the second surface 10R where the recesses 12b are located, and portions on the first surface 10F where the recesses 11b are located correspond to portions on the second surface 10R where the projections 12a are located.

The portions where the projections 11a are located on the first surface 10F and the corresponding recesses 12b are located on the second surface 10R are elements 13. The thin film 10 includes a plurality of elements 13.

With reference to Figs. 1 to 3, specific examples of the structure of the thin film 10 will be described. For ease of understanding, a ratio of the configuration including the elements 13 shown in these drawings may be different from that of the actual structure.

In the thin film 10 shown in the Fig. 1, a plurality of elements 13 having the same shape are regularly arranged. The surface of the elements 13, that is, the surface of the projections 11a and the surface of the recesses 12b are formed as a curved surface, and a portion of the surface of the elements 13 where the top of the projection 11a and the bottom of the recess 12b are located has a curvature. The elements 13 may have, for example, a hemispherical shape, a semi-ellipsoidal shape, or a shape in which an apex of a cone is replaced with a curved surface. The bottom of the recess 11b on the first surface 10F and the top of the projection 12a on the second surface 10R are also formed as a curved surface.

As shown in Fig. 2, the elements 13 may have flat surfaces, and may be pointed toward the apex of the projection 11a. The elements 13 may have, for example, a pyramid shape. In Fig. 2, the bottom of the recess 11b on the first surface 10F and the top of the projection 12a on the second surface 10R are formed as a flat surface.

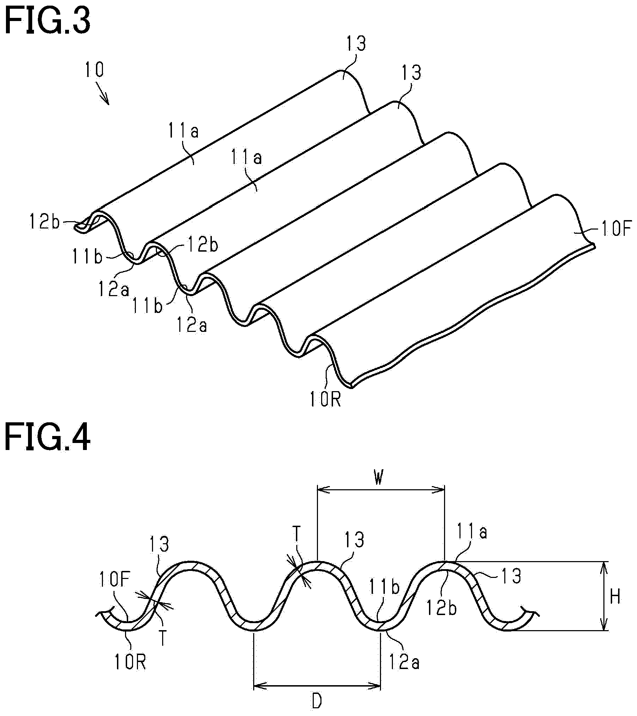

In the thin film 10 shown in the Fig. 3, a plurality of elements 13 extending in one direction are arranged side by side in a direction perpendicular to the extending direction of the elements. The surface of the elements 13 is curved, and the first surface 10F and the second surface 10R have a wavy shape in which undulations are repeated.

The shape of the elements 13 is not limited to the above examples, and may have a conical, frustum, or columnar shape or may have a three-dimensional shape different from those described above. Further, the elements 13 may have a three-dimensional shape having no axis of symmetry. In short, the elements 13 may have a shape in which a portion other than the bottom is surrounded by side walls having a surface formed as at least one of a curved surface and a flat surface. Further, the plurality of elements 13 may include elements 13 having shapes different from each other.

Further, the bottom of the recess 11b on the first surface 10F and the top of the projection 12a on the second surface 10R may be formed as at least one of a curved surface and a flat surface, or the bottom and the top may include both a curved surface and a flat surface.

Moreover, the plurality of elements 13 may also be arranged irregularly. Further, the thickness of the thin film 10 itself may be constant or may not be constant.

Figs. 4 to 7 illustrate examples of a cross-sectional structure of the thin film 10.

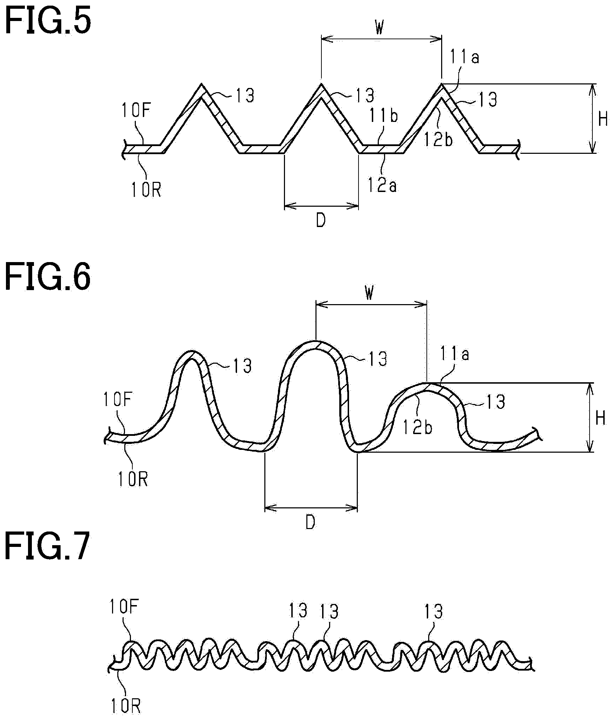

Figs. 4 and 5 illustrate cross-sectional structures in which a plurality of elements 13 having the same shape are arranged at regular intervals. Fig. 4 illustrates an example in which the elements 13 have a curved surface, and the bottom of the recess 11b and the top of the projection 12a are also formed as a curved surface. In the above example, the thin film 10 are curved at the top and the base edge of the projections 11a. In other words, the thin film 10 is repeatedly curved to form the elements 13, which form a concavo-convex structure on the first surface 10F and the second surface 10R.

Fig. 5 illustrates an example in which the elements 13 have flat surfaces, and the bottom of the recess 11b and the top of the projection 12a are also formed as a flat surface. In the above example, the thin film 10 is bent to form corners at the top and the base edge of the projection 11a. In other words, the thin film 10 is repeatedly bent to form the elements 13, which form a concavo-convex structure on the first surface 10F and the second surface 10R.

Fig. 6 illustrates a cross-sectional structure of an example in which a plurality of elements 13 have different shapes and have irregular intervals between the adj acent elements 13. The surface of the elements 13 is formed as at least one of a curved surface and a flat surface. The bottom of the recess 11b and the top of the projection 12a are also formed as at least one of a curved surface and a flat surface. In this case, the thin film 10 is curved and has a curvature or is bent to form corners at the top and the base edge of the projection 11a. The thin film 10 is at least repeatedly curved or bent to form the elements 13, which form a concavo-convex structure on the first surface 10F and the second surface 10R.

As described above, in the present embodiment, the thin film 10 itself is curved to form a concavo-convex structure on the first surface 10F and the second surface 10R. The cross-section of the thin film 10 includes portions where the tops of the projections 11a are located on the first surface 10F and the corresponding bottoms of the recesses 12b are located on the second surface 10R (first portions), and portions where the bottoms of the recesses 11b are located on the first surface 10F and the corresponding tops of the projections 12a are located on the second surface 10R (second portions). The first portions and the second portions are alternately arranged. When the tops of the projections 11a and 12a and the bottoms of the recesses 11b and 12b are formed as a curved surface, the cross-section of the thin film 10 has a wavy shape, in other words, an undulated shape.

In the cross-section of the thin film 10, when portions curved or bent at the top of the projections 11a in the thin film 10 are referred to as convex portions and portions curved or bent at the base edge of the projections 11a are referred to as concave portions, a region between two concave portions that sandwich one convex portion is one element 13.

Preferable ranges of parameters related to the elements 13 will be described below. In the following description, an extending direction of the thin film 10 is defined as a direction parallel to the thin film 10 when the thin film 10 is placed on a flat surface. In this case, the plurality of elements 13 are arranged side by side in the extending direction of the thin film 10. Further, the cross-section of the thin film 10 is a section taken in the direction perpendicular to the plane that is parallel to the extending direction of the thin film 10.

As shown in Fig. 4, an average thickness of the thin film 10 is an average thickness of the film itself, which is an average of the film thicknesses T measured at a plurality of measurement points. The average thickness of the thin film 10 is measured by the following method. First, samples for cross-sectional observation of the thin film 10 are prepared from a plurality of portions of the thin film 10. For example, when the thin film 10 has a rectangular shape in plan view, five samples are prepared from the center and four corners of the thin film 10. Then, the cross-section of each sample is observed using an electron microscope to measure the film thicknesses T at five measurement points, which are evenly distributed within a range of 250 µm across the extending direction of the thin film 10. The film thickness T is a thickness of the film in a direction normal to the first surface 10F at the measurement point. Then, the film thicknesses T at the five measurement points are averaged to obtain an average thickness of each sample, and the average thicknesses of all the samples are averaged to obtain an average thickness of the thin film 10.

The average thickness of the thin film 10 is 0.1 µm or more and 5.0 µm or less. Further, the average thickness of the thin film 10 is preferably 0.1 µm or more and 2.0 µm or less, and more preferably 0.3 µm or more and 1.0 µm or less. When the average thickness of the thin film 10 is not less than the above lower limit, the thin film 10 has strength sufficient to prevent it from being torn when the thin film 10 is lightly touched by hand, which improves ease of handling of the thin film 10 and enhances durability of the thin film 10. When the average thickness of the thin film 10 is not more than the above upper limit, the thin film 10 has good flexibility and improved shape conformability, which provides the thin film 10 with good adhesion to an adherend when adhered to biological tissues.

The mass per unit area of the thin film 10 is preferably 0.1 g/m² or more and 5.0 g/m² or less. The above mass is a mass of a portion of the thin film 10 having an area of 1 m² in plan view of the first surface 10F when viewed in a direction perpendicular to a plane parallel to the extending direction of the thin film 10. The density of the thin film 10 is, for example, 1 g/cm³ or more and 3 g/cm³ or less. When the mass per unit area of the thin film 10 is within the above range, the thickness of the thin film 10 can be prevented from increasing, so the thin film 10 has good flexibility. Since this improves the shape conformability of the thin film 10, the thin film 10 has good adhesion to an adherend.

As shown in Figs. 4 to 6, a distance between the adjacent elements 13 in the extending direction of the thin film 10 is an element interval W. That is, the element interval W is a distance between apexes of the adjacent projections 11a in the extending direction of the thin film 10. When the plurality of elements 13 are regularly arranged, the element interval W is constant or regularly changes in the array of the plurality of elements 13. When the plurality of elements 13 are irregularly arranged, the element interval W irregularly changes in the array of the plurality of elements 13.

When the element interval W between the adjacent elements 13 is large, the bottom of the recess 11b and the top of the projection 12a are likely to be flat, and a proportion of a region having such a flat film shape is likely to increase. In order to prevent the first surface 10F and the second surface 10R from appearing colored and shiny due to interference of light reflected on the first surface 10F and light reflected on the second surface 10R, the proportion of a region having a flat film shape is preferably small. From this viewpoint, the element interval W is preferably 100 µm or less.

Further, the element interval W of 100 µm or less is also preferable from a viewpoint that the surface of the thin film 10 with a larger element interval W tends to appear visually as having a rough texture.

The average height of the elements 13 is an average of heights H of a plurality of elements 13. The average height of the elements 13 is measured by the following method. First, the cross-section of respective samples, which are prepared in the same manner as for measurement of the average thickness of the thin film 10, is observed using an electron microscope to measure the height H of the five elements 13 included in a range of 250 µm across the extending direction of the thin film 10. The height H is a length between the edges of the element 13 in a direction perpendicular to the plane that is parallel to the extending direction of the thin film 10. That is, the height H is a length in a direction perpendicular to the above-mentioned plane from a most protruding point on the first surface 10F in the convex portion constituting the element 13 to a most protruding point on the second surface 10R in the concave portion located at the edge of the element 13. Then, the heights H of the five elements 13 are averaged to obtain an average height of the elements 13 for each sample, and the average heights of the elements 13 for all the samples are averaged to obtain an average height between the elements 13 of the thin film 10.

The average width of the elements 13 is an average of widths D of a plurality of elements 13. The average width of the elements 13 is measured by the following method. First, the cross-section of respective samples, which are prepared in the same manner as for measurement of the average thickness of the thin film 10, is observed using an electron microscope to measure the width D of the five elements 13 included in a range of 250 µm across the extending direction of the thin film 10. The width D is a length between the edges of the element 13 in the extending direction of the thin film 10. That is, the width D is a length in the extending direction of the thin film 10 between most protruding points on the second surface 10R in two adjacent concave portions located at the edges of the element 13. Then, the widths D of the five elements 13 are averaged to obtain an average width of the elements 13 for each sample, and the average widths of the elements 13 for all the samples are averaged to obtain an average width between the elements 13 of the thin film 10.

The average height of the elements 13 is preferably 2 times or more and 100 times or less the average thickness of the thin film 10, and more preferably 5 times or more and 30 times or less the average thickness of the thin film 10. Within the above ranges, the average height of the elements 13 is preferably 2.0 µm or more and 50.0 µm or less, and more preferably 2.0 µm or more and 20.0 µm or less.

When the average height of the elements 13 is not less than the above lower limit, an effect of scattering light due to the concavo-convex structure of the elements 13 can be favorably obtained, and the thin film 10 can be suitably prevented from appearing colored or shiny due to interference of light. When the average height of the elements 13 is not more than the above upper limit, the thin film 10 tends to have good adhesion to an adherend when adhered to biological tissues. Fig. 7 illustrates an example in which the average height of the elements 13 relative to the average thickness of the thin film 10 is smaller than that in Figs. 4 to 6.

A ratio of the average height to the average width of the elements 13 is preferably 0.1 or more and 1.5 or less, and more preferably 0.1 or more and 1.0 or less. When the above ratio is not less than the above lower limit, effects provided by the shape of the thin film 10 of the present embodiment, that is, effects such as scattering of light and an increase in the surface area due to the elements 13 can be favorably obtained. When the above ratio is not more than the above upper limit, the thin film 10 can be easily produced and has good strength and adhesion to an adherend.

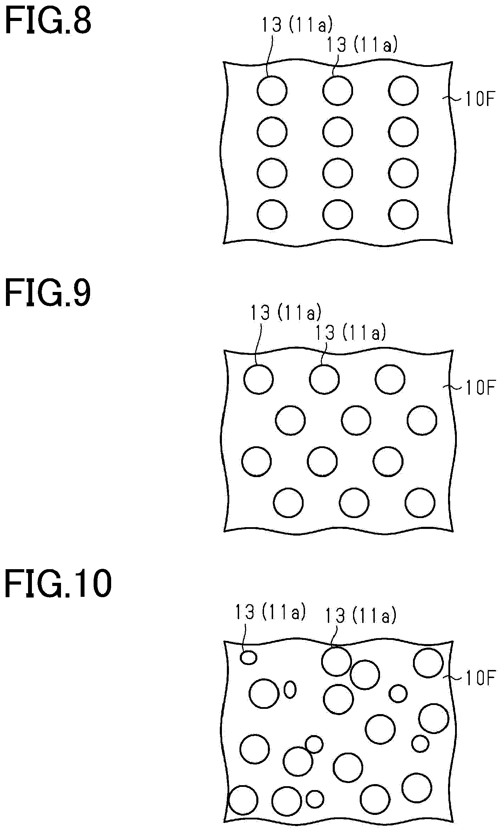

Figs. 8 to 10 illustrate examples of arrangement of the elements 13 in plan view, viewed in a direction perpendicular to a plane parallel to the extending direction of the thin film 10. In Figs. 8 to 10, the elements 13 are illustrated as having a substantially circular outer shape for the sake of convenience, but the outer shape of the elements 13 in plan view is not limited thereto.

Fig. 8 and 9 illustrate examples in which a plurality of elements 13 having the same size are regularly arranged. In Fig. 8, a plurality of elements 13 are located on grid points of a virtual grid formed by grid lines perpendicular to each other. In Fig. 9, a plurality of elements 13 are located on grid points of a virtual grid formed by grid lines obliquely intersecting each other. The element interval W between the elements 13 may differ depending on the extending directions of the grid lines as shown in Fig. 8, or may be the same in two extending directions of the grid lines as shown in Fig. 9.

Fig. 10 illustrates an example in which a plurality of elements 13 are irregularly arranged and have sizes different from each other. When a plurality of elements 13 having the same size are regularly arranged, diffraction of light may occur to cause the first surface 10F and the second surface 10R to appear colored. From the viewpoint of suppressing such a diffraction phenomenon, a plurality of elements 13 preferably have different sizes and are irregularly arranged.

When an area of the entire thin film 10 in plan view described above, that is, an area of the image of the thin film 10 projected onto a plane that is parallel to the extending direction of the thin film 10 is defined as a reference area, the surface area of the first surface 10F is preferably 1.1 times or more and 3.0 times or less the reference area. The surface area of the first surface 10F is an actual area of the first surface 10F including the side surfaces of the concavo-convex structure. Accordingly, the surface area of the first surface 10F is larger than the reference area by an amount corresponding to the area of the side surfaces of the concavo-convex structure.

The reference area may be calculated according to the exterior shape of the thin film 10. For example, when the thin film 10 has a rectangular shape in plan view, the reference area is obtained by multiplying the length of a short side and the length of a long side of the rectangle.

When the elements 13 are formed in a designed shape such as the case where the elements 13 are formed using a mold produced by cutting, the surface area of the first surface 10F may be calculated based on the design values. When the elements 13 are formed in a random shape such as the case where the elements 13 are formed using a mold produced by sandblasting, the surface area of the first surface 10F may be calculated based on the actual measurement values. The actual measurement values can be measured using a microscope having an electric stage for the Z axis, which is the height direction, a confocal laser scanning microscope, a surface roughness meter, an atomic force microscope, or the like.

When the surface area of the first surface 10F is 1.1 times or more the reference area, an effect provided by an increase in the surface area of the first surface 10F due to the elements 13 can be favorably obtained. When the surface area of the first surface 10F is 3.0 times or less the reference area, the thin film 10 has good strength.

According to the thin film 10 of the present embodiment, the thin film 10 itself is curved to form a concavo-convex structure on the first surface 10F and the second surface 10R. Therefore, compared with a case where the thin film 10 has a flat surface on one side and a concavo-convex surface on the other side, the thin film 10 can be provided with a concavo-convex surface while preventing an increase in the thickness of the thin film 10 itself. Accordingly, it is possible to prevent a decrease in shape conformability of the thin film 10 while improving other functions.

Specifically, as described above, since light is scattered due to the concavo-convex structure on the first surface 10F and the second surface 10R, a colored or shiny appearance due to interference of light can be suppressed. Accordingly, the thin film 10 is prevented from being regarded as having poor appearance, and, when the thin film 10 is used for cosmetic applications, it is possible to prevent an adhered portion of the thin film 10 from appearing shiny.

Parameters that are improved by scattering of light due to the above concavo-convex structure include haze. Haze is a parameter indicating the ratio of the diffuse component to the total transmitted light through the thin film 10, and is measured by a method according to JIS K 7136-2000.

The thin film 10 preferably has haze of 7% or more and 65% or less. When the thin film 10 is adhered to the skin and used for assisting in skin care and makeup, the thin film 10 desirably has a high soft focus effect. The soft focus effect refers to properties that blur fine imperfections such as wrinkles, spots, and freckles on the skin surface while allowing the presence of the skin to be recognized via the adhered portion of the thin film 10. The greater the haze, the better the soft focus effect.

When the haze is 7% or more, colored and shiny appearance due to interference of light can be suitably suppressed, and a good soft focus effect can be obtained. Further, when the haze is 65% or less, the thin film 10 is prevented from appearing cloudy and an adhered portion of the thin film 10 is prevented from being conspicuous.

Since the surface area of the first surface 10F and the second surface 10R increases due to the concavo-convex structure compared with a case where the first surface 10F and the second surface 10R are flat, the amount of active ingredients that can be added to the thin film 10 can be increased. Further, due to the concavo-convex structure on the first surface 10F and the second surface 10R, an effect of preventing adhesion can be improved when the thin film 10 is used for an anti-adhesion application, and an effect of promoting proliferation of fibroblasts can be achieved when the thin film 10 is used for a wound treatment application.

Further, when the thin film 10 is used for a cell culture application, the concavo-convex structure on the first surface 10F and the second surface 10R contributes to prevention of an excessive increase in the density of cells to be cultured while preventing a decrease in the characteristics of a thin film suitable as a substrate of cell culture. Accordingly, oxygen and nutrients are easily distributed to the respective cells.

Materials of the thin film 10 will be described below. The thin film 10 may be made of a single thin film, or may be a laminate of a plurality of thin films. The thin film 10 is made of a material having biocompatibility or biodegradability. When the thin film 10 is adhered to the skin, the material of the thin film 10 is preferably a resin having low toxicity, skin irritation, and skin sensitization.

Examples of the material for the thin film 10 include ester resins such as polylactic acid, polyglycolic acid, and polycaprolactone, and copolymer resins thereof. Further, resins for use as film-formers in cosmetics may also be used as the material for the thin film 10. Examples of such resins include acrylic resin, silicone, and copolymer resins thereof, and cellulose derivatives such as cellulose acetate, cellulose acetate propionate, and cellulose acetate butyrate. Further, examples of the material for the thin film 10 include resins that are commonly used as materials for medical products, such as polycarbonate, cycloolefin copolymer, styrene-butadiene elastomer, and polyimide. Examples of the material for the thin film 10 further include proteins such as laminin, peptides, fibronectin, integrin, tenascin, albumin, keratin, collagen, and gelatin, and polysaccharides such as chitin, chitosan, hyaluronic acid, glucomannan, pullulan, dextran, and sacran. Although proteins and polysaccharides may be often vulnerable to water, water resistance of the film can be improved when an ion complex is formed by mixing cationic polymers and anionic polymers or laminating thin films made of these polymers.

Moreover, when the thin film 10 is for use in the living body or as a substrate for cell culture, the thin film 10 preferably has a coating layer made of (methacryloyloxyethyl phosphoryl choline) polymer, poly (2-methoxyethylacrylate), or the like for controlling protein adsorption. Further, when the thin film 10 is used for cell adhesion, the thin film 10 preferably has a coating layer made of proteins such as fibronectin, or inorganic materials such as hydroxyapatite or calcium carbonate.

In addition, when the thin film 10 includes a plurality of layers, each layer may have a composition different from that of the others to perform a function different from that of the others. Accordingly, functions of the thin film 10 can be enhanced or functions can be added to the thin film 10. Examples of these cases will be described below.

When the thin film 10 is formed of a plurality of layers, one of a layer having the first surface 10F and a layer having the second surface 10R is a contact layer that is in contact with an adherend when the thin film 10 is adhered to the adherend, and the other is a non-contact layer. The non-contact layer is an outermost layer exposed to the outside when the thin film 10 is adhered to the adherend.

In a first example, the contact layer includes a material that is likely to cause an interaction such as a hydrogen bond with proteins constituting an adherend which is a biological tissue. Such an interaction occurring between the contact layer and the adherend improves the adhesion between the thin film 10 and the adherend.

The material for the above interaction may be a polymer material that is a main component of the contact layer, or may be added to the material of the contact layer in addition to the main component. Further, the material for the above interaction may be contained at least in the material constituting the surface in contact with the adherend. Therefore, the above material may also be introduced into the contact layer by a surface treatment performed to the contact layer. Examples of the surface treatment include corona treatment, plasma treatment, flame treatment, primer treatment, and UV radiation treatment.

In a second example, water repellency is imparted to the non-contact layer, which is the outermost layer. A polymer material having high hydrophobicity or a water repellent additive may be added to the non-contact layer to impart water repellency to the non-contact layer. Due to the non-contact layer having water repellency, adhesion of external dirt including water droplets or water to the thin film 10 can be suppressed.

In a third example, the thin film 10 is used for cosmetic applications, and cosmetics are applied to the thin film 10 after the thin film 10 is adhered to the skin. That is, cosmetics are applied to the surface of the non-contact layer. In the third example, the non-contact layer contains an oil-soluble additive which is highly miscible with cosmetics. Due to the non-contact layer containing an oil-soluble additive, cosmetics can be easily held on the thin film 10.

The thin film 10 may contain additives depending on the application. As described above, since the surface area of the first surface 10F and the second surface 10R increases due to the concavo-convex structure, the amount of active ingredients of the additives that can be added to the thin film 10 can be increased. Accordingly, functions of the additives can be suitably performed.

For example, for a cosmetic application, examples of additives include moisturizing components, components for colorants or the like that contribute to improvement in design, and cosmetics or cosmetic ingredients used for skin care, such as moisturizing creams and beauty essences. Due to the thin film 10 containing such components, the cosmetic effect produced by the thin film 10 can be improved. Further, examples of the additives include components for measures against foreign body reaction and components for preventing adhesion for an anti-adhesion application, hemostatic components and antibacterial components for a wound treatment application, and adhesive components and aeration components for a cell culture application. The thin film 10 may contain particles, drugs, or the like that function as the above components as an additive added to the thin film 10.

Further, the thin film 10 may also contain the above water repellent additive or layered inorganic compounds as an additive. Due to such additives being added, the thin film 10 does not easily transmit water vapor. Accordingly, when the thin film 10 is adhere to an adherend, water can be easily retained on the surface of the adherend. Therefore, in cosmetic applications, the thin film 10 can be provided with high moisturizing function in addition to the good soft focus effect described above. As the layered inorganic compounds, fragments of clay minerals, for example, smectite group clay minerals such as mica, montmorillonite, saponite, hectorite, and fluorohectorite; kaolin group clay minerals such as kaolinite; magadiite, kenyaite, kanemite, and the like can be used.

Further, the thin film 10 may contain fragrance ingredients as an additive. Accordingly, a thin film 10 emitting fragrance can be obtained. Although fragrance is easily volatilized, the thin film 10 of the present embodiment can contain an increased amount of fragrance ingredients, which causes the fragrance to last longer.

### [Transfer Sheet]

A transfer sheet is used when the thin film 10 is adhered to an adherend which is a biological tissue.

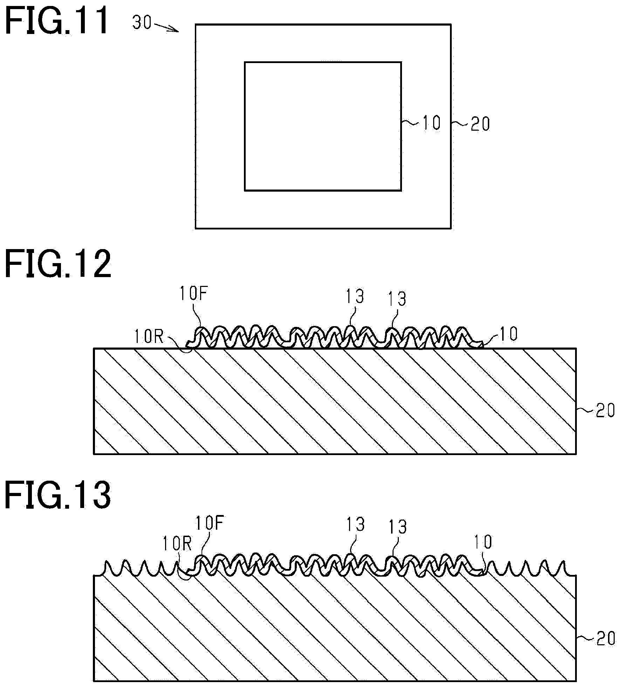

As shown in Fig. 11, a transfer sheet 30 includes the thin film 10, and a support substrate 20 that supports the thin film 10. The exterior shape of the support substrate 20 may be larger than the thin film 10 when viewed in a direction perpendicular to the plane parallel to the extending direction of the thin film 10 as shown in Fig. 11, or may coincide with the exterior shape of the thin film 10. In short, the entire thin film 10 may be supported by the support substrate 20.

A surface of the thin film 10 supported by the support substrate 20, that is, a surface in contact with the support substrate 20 may be the first surface 10F or the second surface 10R. Of the first surface 10F and the second surface 10R, a surface on a side opposite to that in contact with the support substrate 20 is adhered to an adherend.

As shown in Fig. 12, a surface of the support substrate 20 in contact with the thin film 10 may be a flat surface such that a gap is formed in part between the thin film 10 and the support substrate 20. Alternatively, as shown in Fig. 13, a surface of the support substrate 20 in contact with the thin film 10 may have a concavo-convex structure conforming to the thin film 10 such that the thin film 10 and the support substrate 20 are in close contact with each other without a gap being formed. Further, a surface of the support substrate 20 in contact with the thin film 10 may have a concavo-convex structure different from that of the thin film 10 such that a gap is formed in part between the thin film 10 and the support substrate 20. Figs. 12 and 13 illustrate examples in which the second surface 10R of the thin film 10 is in contact with the support substrate 20.

The support substrate 20 has a function of reducing deformation such as wrinkles or folds of the thin film 10 during storage of the transfer sheet 30 and transport of the thin film 10 onto the adherend in use of the transfer sheet 30. Since the thin film 10 is supported by the support substrate 20, ease of handling of the thin film 10 can be increased.

Although materials of the support substrate 20 are not specifically limited, it is preferred that, in use of the transfer sheet 30, releasability of the support substrate 20 from the thin film 10 can be enhanced by physical or chemical stimulus so that the support substrate 20 can be easily removed from the thin film 10.

Examples of the stimulus that changes the releasability of the support substrate 20 include UV radiation, microwave radiation, heating, pressurization, exposure to oxygen, exposure to liquid such as water, and the like. In view of ease of applying the stimulus to the transfer sheet 30, the stimulus is preferably exposure to water. In order to increase the releasability of the support substrate 20 from the thin film 10 by supplying water to the transfer sheet 30, the support substrate 20 preferably has properties of deforming when exposed to water. Deformation includes expansion due to swelling, and dissolution. Examples of the material for the support substrate 20 include cellulose, polyvinyl alcohol, polyvinyl pyrrolidone, polyacrylic acid, and derivatives thereof, proteins, polysaccharides, and polymers containing a large amount of inorganic salts.

Preferably, the support substrate 20 is permeable to liquid and has a concavo-convex surface to form a gap in part between the thin film 10 and the support substrate 20 when it is in contact with the thin film 10. With such a support substrate 20, the support substrate 20 can easily deform when water is supplied to the transfer sheet 30. Further, when water infiltrates between the thin film 10 and the support substrate 20, the support substrate 20 can be easily removed from the thin film 10. Specifically, the support substrate 20 is preferably a woven fabric, a non-woven fabric, a mesh sheet, or a sheet of paper.

Further, a resin sheet that can be sterilized may also be used as the support substrate 20.

### [Method for Producing Thin Film and Transfer Sheet]

An example of a method for producing the thin film 10 and the transfer sheet 30 will be described below. It should be noted that the thin film 10 and the transfer sheet 30 may be produced by a method different from that described below as long as the thin film 10 having the above-mentioned plurality of elements 13 can be formed.

A plurality of elements 13, that is, the concavo-convex structure on the first surface 10F and the second surface 10R are formed using a mold sheet, which is a sheet having a concavo-convex structure on the surface. The concavo-convex shape of the first surface 10F and the second surface 10R conforms to the concavo-convex shape of the above mold.

A concavo-convex structure may be formed on the mold sheet by, for example, cutting with a machine tool or a laser processing machine, photolithography, sandblasting, chemical etching, or forming a plurality of isolated island-like structures using phase separation by blending different polymers. The mold sheet is an original plate made of a metal, or a duplicate plate obtained by duplicating the original plate. When the mold sheet is a duplicate plate, the mold sheet is preferably made of a material that facilitates removal of the mold sheet from the thin film 10. Such a material may be, for example, an olefin-based resin, a silicone-based resin, or a fluorinebased resin.

As the method for producing a thin film 10 by using a mold sheet, a first method and a second method will be described below. In the first method, a liquid material containing a material of the thin film 10 is supplied onto a mold sheet, and the liquid material is then cured to form a thin film 10 having a concavo-convex structure. Specifically, the method may use a solution casting method, in which the above liquid material, prepared as a solution containing a material of the thin film 10, is supplied onto a mold sheet by using various coating methods, or a melt extrusion method, in which the above liquid material, prepared by dissolving a resin which is a material of the thin film 10, is supplied onto a mold sheet by extrusion.

In the solution casting method, the solution is prepared by dissolving or dispersing the material of the thin film 10 in an appropriate solvent. Various coating methods can be used, including direct gravure, reverse gravure, small diameter reverse gravure, Mayer coating, die, curtain, spray or spin coating, screen printing, comma, knife, gravure offset, and roll coating. When the thin film 10 is formed into a predetermined pattern, coating methods including direct gravure, spraying, screen printing, and gravure offset can be suitably used. The film thickness of the thin film 10 can be controlled by the solid content ratio in the solution and the coating method to be used. The solution supplied onto the mold sheet is dried and solidified to form a thin film 10.

In the melt extrusion method, a resin is heated and sheared by a screw to melt the resin, and the resin is extruded onto a mold sheet. Then, the resin on the mold sheet is cooled and solidified to form a thin film 10. The film thickness of the thin film 10 can be controlled by the relationship between the amount of resin to be extruded and the take-up speed, or the stretching ratio.

In the second method, after a thin film is prepared as a material of the thin film 10, the thin film is softened by heat, solvent, or the like. Then, a mold sheet is pressed against the thin film to form a thin film 10 having a concavo-convex structure. The mold sheet can be pressed against the thin film by using pneumatic or hydraulic pressure.

In both the first and second methods, the film thickness of the thin film 10 is made smaller than the size of the concavo-convex structure of the mold sheet in the thickness direction, that is, the depth of recesses and the height of projections. Accordingly, the entire thin film 10 undergoes bending and forms a concavo-convex structure on the first surface 10F and the second surface 10R, rather than only on one of the surfaces.

Further, the recesses or projections on the mold sheet may include recesses or projections having a size in the thickness direction smaller than the film thickness of the thin film 10. Such recesses or projections, when pressed against the thin film 10, form a concavo-convex structure that is located on only one side of the thin film 10. That is, the concavo-convex structure on the first surface 10F and the second surface 10R may include recesses or projections that do not constitute an element 13.

The thin film 10 formed on the mold sheet is transferred onto the support substrate 20 to form a transfer sheet 30. Various transfer methods may be used to transfer the thin film 10 from the mold sheet to the support substrate 20. When the above production methods are used, a surface of the support substrate 20 does not conform to the concavo-convex structure on the thin film 10, and a gap is formed in part between the thin film 10 and the support substrate 20.

When the average thickness of the thin film 10 is small, or the ratio of the average height to the average width of the elements 13 is large, the strength of the thin film 10 decreases, causing the thin film 10 to be easily torn when the mold sheet is removed. In such cases, a lubricant or a mold release agent such as oil or silicone can be applied to a surface of the mold sheet to enhance the releasability of the mold sheet from the thin film 10.

Further, in order to prevent the support substrate 20 from being detached from the thin film 10 before use of the transfer sheet 30, temporary fixation between the thin film 10 and the support substrate 20 may be performed. For the temporary fixation, for example, partial thermal fusion bonding, adhesion with a biocompatible adhesive, or the like may be used.

When the thin film 10 adheres to an adherend with an adhesion force larger than that required for removing the mold sheet from the thin film 10, the mold sheet may be used as a support substrate 20. Such cases include a case where an adherend has an adhesive surface, and a case where a substance such as an adhesive liquid is supplied to adhere the thin film 10 to an adherend. When the mold sheet is used as a support substrate 20, a surface of the support substrate 20 conforms to the concavo-convex structure of the thin film 10 and is in close contact with the thin film 10.

### [Method for Adhering Thin Film]

The thin film 10 is adhered to an adherend by transferring the thin film 10 in the transfer sheet 30 from the support substrate 20 to the adherend.

In a case where the support substrate 20 has releasability from the thin film 10, which is enhanced when physical or chemical stimulus is applied, the stimulus can trigger an increase in the releasability of the support substrate 20 from the thin film 10 only in use of the transfer sheet 30. Therefore, before use of the transfer sheet 30, deformation of the thin film 10 due to the support substrate 20 can be suitably suppressed.

A specific example in which the above stimulus is exposure to liquid such as water will be described below. First, liquid such as water, alcohol, or oil is supplied onto an adherend, and the transfer sheet 30 is then placed on the adherend with the thin film 10 being in contact with the adherend. As the liquid on the adherend penetrates to the support substrate 20, the releasability of the support substrate 20 increases. When the support substrate 20 is removed from the thin film 10, the thin film 10 is transferred from the support substrate 20 to the adherend. Further, a body fluid present on a surface of the adherend may also be used as the liquid described above. Alternatively, the liquid may also be supplied to the transfer sheet 30 after the transfer sheet 30 is placed on the adherend.

The method for adhering the thin film 10 is not limited to that described above, and may include the following examples. In a first example, in transfer of the thin film 10 to the adherend, water, alcohol, or the like is interposed between an adherend and the thin film 10. As the interposed water or alcohol naturally disappears, the thin film 10 is adhered to the adherend. In a second example, after an adhesive substrate in a form of a liquid or cream is applied onto an adherend, the transfer sheet 30 is placed on the adherend. Then, the support substrate 20 is removed. In a third example, while the transfer sheet 30 is floated on water, the support substrate 20 is removed. The thin film 10 is placed on an adherend when the adherend is pulled out from water. Then, as the water naturally disappears, the thin film 10 is adhered to the adherend. In a fourth example, the thin film 10 is transferred to a frame-shaped structure so that the thin film 10 is supported by the structure. The thin film 10 positioned in the frame is then provided on an adherend to thereby adhere the thin film 10 to the adherend.

The thin film 10 may be used to protect a region on the adherend where a semi-solid agent is applied. The semi-solid agent may be ointment or cream, and includes components that perform predetermined functions in medical applications or cosmetic applications.

After the semi-solid agent is applied to a surface of the adherend, the thin film 10 is adhered to the region where the semi-solid agent is applied so that the applied region is covered with the thin film 10. As the region where the semi-solid agent is applied is thus covered with the thin film 10, it is possible to suppress stickiness of the region due to the applied semi-solid agent, and make the applied region inconspicuous by suppressing a glossy appearance of the applied semi-solid agent. Since the thin film 10 of the present embodiment has a good soft focus effect as described above, a glossy appearance of the region where the semi-solid agent is applied can be suitably suppressed.

### [Examples]

The thin film and the transfer sheet described above will be described by using specific examples and comparative examples.

### (Example 1)

Polypropylene in an amount of 100 g/m² was supplied onto a mold roll having a concavo-convex structure on a surface by a melt extrusion method to prepare a mold sheet.

Poly-DL-lactic acid was dissolved in ethyl acetate to prepare a solution having a solid content of 10 wt%. The solution was applied to a mold sheet by a slot die coating method, and then dried with hot air to form a thin film. The thin film had a dry thickness of 0.1 µm. The thin film on the mold sheet was transferred onto a support substrate made of a non-woven fabric. Thus, a transfer sheet of Example 1 was obtained.

In the thin film of Example 1, the average height of the elements was 2.0 µm, a ratio of the average height to the average width of the elements was 1.0, and a ratio of the surface area of the first surface to the reference area was 2.0. These parameters were calculated based on the configuration of the concavo-convex structure on the mold roll.

### (Example 2)

A thin film and a transfer sheet of Example 2 were obtained in the same manner as in Example 1, except that the configuration of the concavo-convex structure on the mold roll was modified. In the thin film of Example 2, the average height of the elements was 2.0 µm, a ratio of the average height to the average width of the elements was 0.5, and a ratio of the surface area of the first surface to the reference area was 1.4. These parameters were calculated based on the configuration of the concavo-convex structure on the mold roll.

### (Example 3)

A thin film and a transfer sheet of Example 3 were obtained in the same manner as in Example 1, except that the configuration of the concavo-convex structure on the mold roll was modified. In the thin film of Example 3, the average height of the elements was 20.0 µm, a ratio of the average height to the average width of the elements was 1.0, and a ratio of the surface area of the first surface to the reference area was 2.0. These parameters were calculated based on the configuration of the concavo-convex structure on the mold roll.

### (Example 4)

A thin film and a transfer sheet of Example 4 were obtained in the same manner as in Example 1, except that the configuration of the concavo-convex structure on the mold roll was modified. In the thin film of Example 4, the average height of the elements was 20.0 µm, a ratio of the average height to the average width of the elements was 0.5, and a ratio of the surface area of the first surface to the reference area was 1.4. These parameters were calculated based on the configuration of the concavo-convex structure on the mold roll.

### (Example 5)

A thin film and a transfer sheet of Example 5 were obtained in the same manner as in Example 1, except that the dry thickness of the thin film was 1.5 µm. In the thin film of Example 5, the average height of the elements was 2.0 µm, a ratio of the average height to the average width of the elements was 1.0, and a ratio of the surface area of the first surface to the reference area was 2.0. These parameters were calculated based on the configuration of the concavo-convex structure on the mold roll.

### (Example 6)

A thin film and a transfer sheet of Example 6 were obtained in the same manner as in Example 1, except that the configuration of the concavo-convex structure on the mold roll was modified and the dry thickness of the thin film was 1.5 µm. In the thin film of Example 6, the average height of the elements was 2.0 µm, a ratio of the average height to the average width of the elements was 0.5, and a ratio of the surface area of the first surface to the reference area was 1.1. These parameters were calculated based on the configuration of the concavo-convex structure on the mold roll.

### (Example 7)

A thin film and a transfer sheet of Example 7 were obtained in the same manner as in Example 1, except that the configuration of the concavo-convex structure on the mold roll was modified and the dry thickness of the thin film was 1.5 µm. In the thin film of Example 7, the average height of the elements was 20.0 µm, a ratio of the average height to the average width of the elements was 1.5, and a ratio of the surface area of the first surface to the reference area was 3.0. These parameters were calculated based on the configuration of the concavo-convex structure on the mold roll.

### (Example 8)

A thin film and a transfer sheet of Example 8 were obtained in the same manner as in Example 1, except that the configuration of the concavo-convex structure on the mold roll was modified and the dry thickness of the thin film was 1.5 µm. In the thin film of Example 8, the average height of the elements was 20.0 µm, a ratio of the average height to the average width of the elements was 0.5, and a ratio of the surface area of the first surface to the reference area was 1.4. These parameters were calculated based on the configuration of the concavo-convex structure on the mold roll.

### (Example 9)

A thin film and a transfer sheet of Example 9 were obtained in the same manner as in Example 1, except that the configuration of the concavo-convex structure on the mold roll was modified and the dry thickness of the thin film was 3.0 µm. In the thin film of Example 9, the average height of the elements was 5.0 µm, a ratio of the average height to the average width of the elements was 1.0, and a ratio of the surface area of the first surface to the reference area was 2.0. These parameters were calculated based on the configuration of the concavo-convex structure on the mold roll.

### (Example 10)

A thin film and a transfer sheet of Example 10 were obtained in the same manner as in Example 1, except that the configuration of the concavo-convex structure on the mold roll was modified and the dry thickness of the thin film was 3.0 µm. In the thin film of Example 10, the average height of the elements was 20.0 µm, a ratio of the average height to the average width of the elements was 1.0, and a ratio of the surface area of the first surface to the reference area was 2.0. These parameters were calculated based on the configuration of the concavo-convex structure on the mold roll.

### (Comparative Example 1)

A thin film and a transfer sheet of Comparative Example 1 were obtained in the same manner as in Example 1, except that the dry thickness of the thin film was 0.05 µm. In the thin film of Comparative Example 1, the average height of the elements was 2.0 µm, a ratio of the average height to the average width of the elements was 1.0, and a ratio of the surface area of the first surface to the reference area was 2.0. These parameters were calculated based on the configuration of the concavo-convex structure on the mold roll.

### (Comparative Example 2)

A thin film and a transfer sheet of Comparative Example 2 were obtained in the same manner as in Example 1, except that the configuration of the concavo-convex structure on the mold roll was modified and the dry thickness of the thin film was 5.5 µm. In the thin film of Comparative Example 2, the average height of the elements was 8.0 µm, a ratio of the average height to the average width of the elements was 1.0, and a ratio of the surface area of the first surface to the reference area was 2.0. These parameters were calculated based on the configuration of the concavo-convex structure on the mold roll.

### (Comparative Example 3)

A thin film and a transfer sheet of Comparative Example 3 were obtained in the same manner as in Example 1, except that the configuration of the concavo-convex structure on the mold roll was modified and the dry thickness of the thin film was 5.5 µm. In the thin film of Comparative Example 3, the average height of the elements was 20.0 µm, a ratio of the average height to the average width of the elements was 1.0, and a ratio of the surface area of the first surface to the reference area was 2.0. These parameters were calculated based on the configuration of the concavo-convex structure on the mold roll.

### (Comparative Example 4)

Poly-DL-lactic acid was dissolved in ethyl acetate to prepare a solution having a solid content of 10 wt%. The solution was applied to a biaxially stretched polypropylene film by a slot die coating method, and then dried with hot air to form a thin film. The thin film had a dry thickness of 0.1 µm. The thin film on the mold sheet was transferred onto a support substrate made of a non-woven fabric. Thus, a transfer sheet of Comparative Example 4 was obtained. In Comparative Example 4, the thin film does not have a concavo-convex structure on either surface.

### (Comparative Example 5)

A thin film and a transfer sheet of Comparative Example 5 were obtained in the same manner as in Comparative Example 4, except that the dry thickness of the thin film was 3.0 µm. In Comparative Example 5, the thin film does not have a concavo-convex structure on either surface.

### (Evaluation Method)

### <Adhesion>

The skin of the arm was moistened with water, and the transfer sheets of the respective examples and comparative examples were placed with the thin film being in contact with the arm. Then, the support substrate was gently removed so that the thin film was transferred onto the skin of the arm. The edge of the thin film was rubbed with a finger. The case where the edge was not peeled was evaluated as "good," the case where the edge was slightly peeled was evaluated as "fair," and the case where the edge was obviously peeled was evaluated as "poor."

### <Appearance>

The appearance of the thin films of the respective examples and comparative examples were observed. The case where colors or glossy luster due to interference of light was observed on the surface was evaluated as "poor," and the case where colors or luster due to interference of light was not observed on the surface was evaluated as "good."

### <Soft Focus Effect>

After water was supplied onto a surface of a 1 mm thick float glass, the transfer sheets of the respective examples and comparative examples were placed with the thin film being in contact with the surface of the float glass. Then, the support substrate was gently removed to prepare a sample in which the thin film was adhered to the float glass.

The sample was provided on a surface of BIOSKIN, manufactured by Beaulax Co., Ltd., on which artificial wrinkles were formed. The appearance of the artificial wrinkles via the sample was evaluated to evaluate the soft focus effect in 3 stages. The case where the soft focus effect was high, that is, where almost no artificial wrinkles were observed was evaluated as "good," the case where soft focus effect was moderate, that is, where artificial wrinkles were blurred as compared with a case where no sample was provided was evaluated as "fair," and the case where soft focus effect was low, that is, where artificial wrinkles were observed as clearly as in a case where no sample was provided was evaluated as "poor."

### (Evaluation Results)

Table 1 shows the average thickness of the thin film, the average height of the elements, the ratio of the average height to the average width of the elements, the ratio of the surface area of the first surface to the reference area, as well as the evaluation results for adhesion, appearance, and soft focus effect for the respective examples and comparative examples.

**[Table 1]**

| | Average thickness (µm) | Average height (µm) | Average height/ Average thickness | Surface area/ Reference area | Adhesi on | Appear ance | Soft Focus Effect |
|---|---|---|---|---|---|---|---|
| Example 1 | 0.1 | 2.0 | 1.0 | 2.0 | good | good | fair |
| Example 2 | 0.1 | 2.0 | 0.5 | 1.4 | good | good | fair |
| Example 3 | 0.1 | 20.0 | 1.0 | 2.0 | good | good | good |
| Example 4 | 0.1 | 20.0 | 0.5 | 1.4 | good | good | good |
| Example 5 | 1.5 | 2.0 | 1.0 | 2.0 | good | good | fair |
| Example 6 | 1.5 | 2.0 | 0.5 | 1.1 | good | good | fair |
| Example 7 | 1.5 | 20.0 | 1.5 | 3.0 | good | good | good |
| Example 8 | 1.5 | 20.0 | 0.5 | 1.4 | good | good | good |
| Example 9 | 3.0 | 5.0 | 1.0 | 2.0 | fair | good | fair |
| Example 10 | 3.0 | 20.0 | 1.0 | 2.0 | fair | good | good |
| Comparativ e Example 1 | 0.05 | 2.0 | 1.0 | 2.0 | good | good | poor |
| Comparativ e Example 2 | 5.5 | 8.0 | 1.0 | 2.0 | poor | good | fair |
| Comparativ e Example 3 | 5.5 | 20.0 | 1.0 | 2.0 | poor | good | good |
| Comparativ e Example 4 | 0.1 | 0.0 | 0.0 | 1.0 | good | poor | poor |
| Comparativ e Example 5 | 3.0 | 0.0 | 0.0 | 1.0 | fair | poor | poor |

As seen from Table 1, in Examples 1 to 10, the adhesion, appearance, and soft focus effect are all evaluated as being moderate or higher. On the other hand, in Comparative Examples 2 and 3, having a large average thickness, the adhesion is low, and in Comparative Examples 4 and 5, having no concavo-convex structure on a surface, the appearance and soft focus effect are evaluated as being low. Further, in Comparative Example 1, having a significantly small average thickness, the soft focus effect is evaluated as being low. The reason for this seems to be that the thin film, having extremely small thickness, fails to exhibit an effect of concealing wrinkles. Therefore, as in Examples 1 to 10, in which the film has an average thickness of 0.1 µm or more and 5.0 µm or less and includes a concavo-convex structure on a surface formed by bends within the film, it is possible to prevent a decrease in the shape conformability to a surface shape of the adherend, which is the characteristics due to a small film thickness, and improve the appearance and soft focus effect.

As seen from the comparison among Examples 1 to 10, there is a tendency that the adhesion increases with a decrease in the average thickness. Further, there is a tendency that the soft focus effect increases with an increase in the average height of the elements.

As an evaluation of the ease of producing the thin film, a surface of the thin film on the support substrate was observed using an optical microscope at a magnification of 50 times for the respective examples and comparative examples. As a result, in Comparative Example 1, it was clearly observed that the thin film was broken or torn by visual inspection. In Examples 1, 3, 5, and 7, it was observed by an optical microscope that the thin film was slightly broken or torn, and in Examples 2, 4, 6, 8 to 10 and Comparative Examples 2 to 5, it was observed by an optical microscope that the thin film was not broken or torn. Therefore, it was found that a thin film having an average thickness of 0.1 µm or more has good strength and is easily produced. Further, when the thin film was slightly broken or torn, such breakage or torn may be preferred for improvement in air permeability of the thin film.

As described in the above embodiments and examples, according to the above thin film and the transfer sheet, the effects listed below can be achieved.
(1) The thin film 10 has an average thickness of 0.1 µm or more and 5.0 µm or less, and includes a plurality of elements 13. With this configuration, compared with a case where a concavo-convex surface is formed only on the first surface 10F, portions of the thin film 10 where the projections 11a are located on the first surface 10F are prevented from increasing in film thickness. Accordingly, the thin film 10 can have a concavo-convex structure on the surfaces while preventing a decrease in the characteristics due to a small film thickness. Due to the concavo-convex structure provided on the surface, light can be scattered and the surface area can be increased. Accordingly, the functions of the thin film 10 can be improved. For example, it is possible to suppress a colored or shiny appearance of the thin film due to interference of light while preventing a decrease in shape conformability of the thin film, and improve the soft focus effect.
(2) The cross-section of the thin film 10 includes: portions where the tops of the projections 11a are located on the first surface 10F and the corresponding bottoms of the recesses 12b are located on the second surface 10R; and portions where the bottoms of the recesses 11b are located on the first surface 10F and the corresponding tops of the projections 12a are located on the second surface 10R. The first portions and the second portions are alternately arranged. That is, since the thin film 10 itself is repeatedly curved to form a concavo-convex structure on the first surface 10F and the second surface 10R, it is possible to obtain an effect provided by a surface having a concavo-convex structure in a wide area of the thin film 10 while preventing a decrease in the characteristics due to a small film thickness.
(3) The element interval W between the elements 13 adjacent to each other is 100 µm or less, the average height of the elements 13 is 2.0 µm or more and 50.0 µm or less, and the ratio of the average height to the average width of the elements 13 is 1.5 or less. With this configuration, the shape conformability, strength, and soft focus effect of the thin film 10 can be favorably obtained, and the thin film 10 can be suitably prevented from appearing colored or shiny due to interference of light.
(4) The surface area of the first surface 10F is 1.1 times or more and 3.0 times or less the reference area. With this configuration, the thin film 10 has good strength, and an effect produced by an increase in the surface area of the thin film 10 can be favorably obtained.
(5) Since the thin film 10 is made of a material having biocompatibility or biodegradability, the thin film 10 has increased suitability for use in applications in which the thin film 10 is adhered to biological tissues and for use as a substrate for cell culture.
(6) In the transfer sheet 30, since the thin film 10 is supported by the support substrate 20, the thin film 10 is prevented from deforming, which improves the ease of handling of the thin film 10.
(7) When the support substrate 20 is one of a woven fabric, a non-woven fabric, a sheet of paper, and a mesh sheet, the support substrate 20 is permeable to liquid. Accordingly, when the support substrate 20 is exposed to liquid such as water, the liquid is easily distributed into the support substrate 20. Therefore, when the support substrate 20 is configured to deform when exposed to liquid, the deformation is promoted and the releasability of the support substrate 20 from the thin film 10 is suitably improved.

### [Reference Signs List]

- 10: Thin film
- 10F: First surface
- 10R: Second surface
- 11a: Projection
- 11b: Recess
- 12a: Projection
- 12b: Recess
- 13: Element
- 20: Support substrate
- 30: Transfer sheet

## Claims

1. A thin film comprising:
a first surface; and a second surface located on a side of the thin film opposite to that on which the first surface is located, wherein
the thin film has an average thickness of 0.1 µm or more and 5.0 µm or less,
the first surface and the second surface each have a concavo-convex structure,
the thin film includes an element, which is a portion where a projection is located on the first surface and a corresponding recess is located on the second surface, and
the thin film includes a plurality of the elements.

2. The thin film according to claim 1, wherein
a cross-section of the thin film includes:
a first portion, which is a portion where a top of a projection is located on the first surface and a corresponding bottom of a recess is located on the second surface; and
a second portion, which is a portion where a bottom of a recess is located on the first surface and a corresponding top of a projection is located on the second surface, the first portion and the second portion being alternately arranged.

3. The thin film according to claim 1 or 2, wherein
an interval between the elements adjacent to each other is 100 µm or less,
an average height of the plurality of elements is 2.0 µm or more and 50.0 µm or less, and
a ratio of the average height to an average width of the plurality of elements is 1.5 or less.

4. The thin film according to any one of claims 1 to 3, wherein, when an area of the thin film in plan view as viewed in a direction perpendicular to a plane parallel to an extending direction of the thin film is defined as a reference area, the first surface has a surface area of 1.1 times or more and 3.0 times or less the reference area.

5. The thin film according to any one of claims 1 to 4, wherein the thin film is made of a material having biocompatibility or biodegradability.

6. A transfer sheet comprising:
the thin film according to any one of claims 1 to 5; and
a support substrate that supports the thin film.

7. The transfer sheet according to claim 6, wherein the support substrate is one of a woven fabric, a non-woven fabric, a sheet of paper, and a mesh sheet.
